(19) Europäisches Patentamt
European Patent Office
Office européen des brevets

(11) Publication number: 0 298 065 A2

# (12) EUROPEAN PATENT APPLICATION

(21) Application number: 88850229.1

(51) Int. Cl.⁴: A 61 K 31/22

(22) Date of filing: 23.06.88

(30) Priority: 29.06.87 SE 8702674

(43) Date of publication of application: 04.01.89 Bulletin 89/01

(84) Designated Contracting States: BE CH DE FR GB IT LI NL SE

(71) Applicant: BEROL KEMI AB
Box 851
S-444 01 Stenungsund 1 (SE)

(72) Inventor: Allenmark, Stig
Östra Björnvägen 4
S-430 41 Kullavik (SE)

Lindstedt, Magnus
Siriusgatan 40
S-415 22 Göteborg (SE)

Edebo, Lars
Dybecksgatan 10
S-412 70 Göteborg (SE)

(74) Representative: Onn, Thorsten et al
AB STOCKHOLMS PATENTBYRA Box 3129
S-103 62 Stockholm (SE)

## (54) Prophylactic agent for controlling venereal diseases.

(57) A prophylactic agent for controlling venereal diseases. As an active microbicidal component, the agent has an ammonium compound having hydrolyzable ester group.



Bundesdruckerei Berlin

## Description

### PROPHYLACTIC AGENT FOR CONTROLLING VENEREAL DISEASES

The present invention refers to a prophylactic agent for controlling venereal diseases, said agent containing as an active microbicidal component an ammonium compound having a hydrolyzable ester bond.

Since a long time it is known that a number of venereal diseases, such as gonorrhoea and chlamydia, are spread by sexual contacts. These diseases are caused by bacteria. In later years, also venereal diseases caused by virus, such as aids and herpes, having got increasing attention. For prophylactic purposes are recommended the use of condoms, foams, ointments, gels and other products, on which have been applied or which contain microbicidal compounds. The microbicidal compounds which have been used, however, have the disadvantage that they give a persistent irritation after use.

Amphipilic compounds of the quaternary ammonium type have for a long time been known to exhibit a strong antimicrobial activity, the disclosure by Domagk in 1935 having great impact on the development of this field. (G. Domagk, Dtsch. Med. Wochenschr. 61, 829 (1935). It has become evident, however, from studies of acute as well as cronic toxicity, that these compounds may give raise to skin irritation and hypersensitivity, and therefore are not quite recommendable for certain applications.

Therefore, it is an object of this invention to find antimicrobial compounds which do not have the drawback mentioned above. Another object of the invention is to permit a rapid inactivation by a contrallable degradation of the antimicrobial compound. Still another object is that the degradation will take place at a pH value from about 6 to about 8.5 which is a pH value that does not give raise to skin irritation and the like. Also, a still further object is to provide a prophylactic agent for controlling veneral diseases caused by bacteria or virus and which contains or has applied to it one or more antimicrobial compounds which do not exhibit the drawback mentioned above.

It has now been shown that the above objects are met by a prophylactic agent which contains or has been treated with a long-chain ammonium compound having the general formula

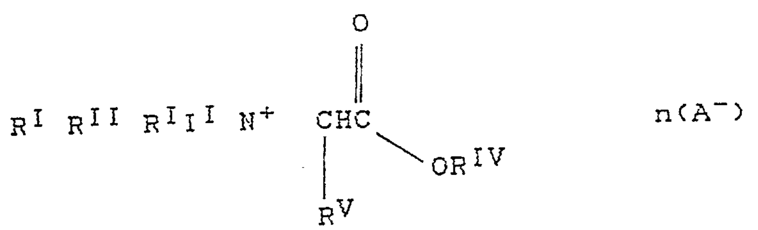

I

wherein $R^I$, $R^{II}$ and $R^{III}$ are hydrogen or lower alkyl groups, and $R^{IV}$ is a long chain alkyl group having 10 to 18 carbon atoms, $R^V$ is hydrogen or a group having the formula $R^{VI}\ N^+H_3$, wherein $R^{VI}$ is an alkylene group having 3 to 4 carbon atoms, A is a monovalent counter ion, and n is the number of cationic groups in the compound, shows a good activity against microorganisms which cause a number of the most common venereal diseases, such as gonorrhoea and chlamydia. Especially surprising and valuable is the fact that the ammonium compound also has a good effect on viruses which cause venereal diseases. Examples of such viruses are herpes simplex virus, human immunodeficiency virus and human papilloma virus. By this it becomes possible also to control aids and herpes.

It is important that the ester bond of the ammonium compound is hydrolyzable at a neutral pH or above. The pH of the vagina is low, however, usually within the range 2-4, and within this range the ammonium compounds are stable and are able to kill bacteria and viruses i the vaginal lumen. At the vaginal wall, however, the pH value increases significantly, and when the ammonium compound gets into contact with the tissues of the wall, its ester bond is hydrolyzed and the ammonium compound is transformed into inactive, harmless products, whereby a remaining microbicidal irritation after use of the prophylatic agent of the invention is avoided. This is a very important feature when the compounds are to be used in prophylactic agents against venereal diseases.

The lower alkyl groups $R^I$, $R^{II}$ and $R^{III}$ preferably are alkyl groups having 1-4 carbon atoms, and most preferably they are methyl groups. Preferably $R^{IV}$ is a straight chain alkyl group. The counter ion A is usually a halide ion, such as chloride, or $HSO_4^-$.

Examples of lower alkyl groups are methyl, ethyl, propyl, isopropyl, butyl, secondary butyl and isobutyl, but ethyl and especially methyl are preferred. The long-chain alkyl group $R^{VI}$ can be decyl, dodecyl, dodecenyl, tetradecyl, hexadecyl, oktadecyl, oktadecenyl, oktadecadienyl and oktadecatrienyl. Among these groups, the saturated groups are especially preferred.

Examples of preferred ammonium compounds are compounds having the formulae

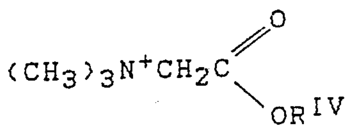 A- II

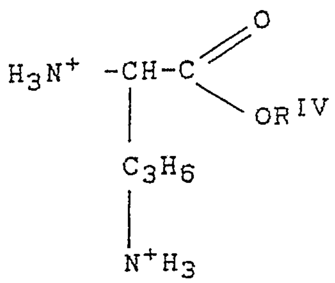 2A- III

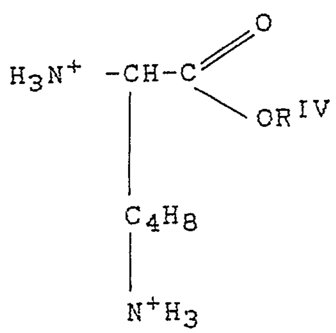 2A- IV

wherein $R^{IV}$ and A have the meanings stated above.

The ammonium compounds described above have previously been disclosed in the chemical litterature (I. Métayer, Ann. pharm. franc. 10, 435, 439 (1952) and they have also been shown to have antibacterial properties (A.E. Epstein et al., Khim. Farm. Zh. 14, 23 (1980) and Teruaki Nakamiya et al., Ferment. Technol. Vol. 54 nr. 6, p. 369-376 (1976). None of these reports discloses that the ammonium compounds in question have a microbicidal effect against bacteria and viruses which cause venereal diseases. Also, there is not disclosure that the compounds hydrolyze rapidly at pH values of about 6 to 8.5, forming non-irritating hydrolysis products.

When an antimicrobial agent is used at appropriate concentration levels, the time needed for full antimicrobial effect is generally fairly short. In most practical situations the antimicrobial agenst is also administrated in a very large excess. Therefore, in many applications the toxic compound is active for a long time after it has exerted its antimicrobial action, which is both undesirable und unneccesary. The invention discloses a principle, according to which the antimicrobial compound loses its biological activity and toxic action by a hydrolytic reaction. The rate of hydrolysis can be regulated by small changes of pH and electrolyte concentration leading to harmless end products within the physiologically suitable pH range from about 6 to about 8.5.

The solubility of the esters in polar organic solvents, such as lower alcohols, and alochol/water mixtures is of the order 1 g/ml. Such formulations are highly stable and allow long-term storage.

The esters are highly active against a broad spectrum of Gram positive as well as Gram negative bacteria and examples of test data are given below. An important and quite unexpected feature of the invention is also that the compounds have pronounced effect on virus which causes venereal diseases.

The temporary desinfecting effect of the esters may be used in hygienic products such as diapers, tampons, masks, napkins, and cosmetic powders. It may also be employed as disinfectants, e.g. in bubble baths, swimming pools, mouthwashes; and for treatment of packages, food etc. The esters can be applied on webs and garments by dipping into or spraying a slightly acid, aqueous solution of the ester followed by a rapid drying. Cellulosic fibers may be impregnated by adding the esters to the cellulose pulp slurry in the head-box. When used in dry form the esters may have the form of a tablet, for example an effervescent tablet. In an important application of the invention, the esters are applied to or incorporated into prophylactic agents for preventing the spreading of venereal diseases.

The esters may be prepared by conventional methods by reacting a chloro- or bromoacetyl halide with an

alcohol in a halogenated hydrocarbon to an alkyl chloro- oc bromoacetate. This ester is then converted into the ester of a betaine by a reaction with gaseous trialkyalamine in a solvent such as acetone or toluene.

The ammonium compounds can in a way known per se be mixed into or applied to agents for controlling the spreading of venereal diseases. Such agents can be sprays, ointments, gels, foams, condoms and vaginal pads. As the ammonium compounds are hydrolyzed at neutral and basic pH, they are usually added in the form of an acid aqueous solution, or alternatively as a solution based on an organic solvent. When the prophylatic agent is a condom or vaginal pad, the ammonium compound is applied by dipping the prophylatic agent in or spraying it with a solution containing the ammonium compound and subsequent drying. If the prophylatic agent is aqueous, its pH value should be lower than 5.

The invention is further illustrated by the following examples.

Example 1

The bactericidal effect of three esters having the following formula

$(CH_3)_3 \overset{\oplus}{N} CH_2CO_2CH_2(CH_2)_p CH_3$

where p was 10, 12 and 14 respectively were determined and compared with cetyltrimetylammonium bromide (CTAB), a well-known bactericide. The effect was measured as the concentration needed for reducing the number of viable bacteria by a factor of $10^5$ using E. coli (NCTC 10418) as test organism during 5 minutes contact time. The results are shown in the Table below.

TABLE 1

| Compound p | Concentration ppm |
|---|---|
| 10 | 50 |
| 12 | 12.5 |
| 14 | >100 |
| CTAB | 12.5 |

From the result it is evident that the testers haven an appropriate bactericidal effect.

Example 2

The activity towards a series of microorganisms was determined for the compound in Example 1 having totally 14 carbon atoms in its alkyl chain (p = 12). Relevant test data are given in the Table below. The activity is expressed as log (cfu/ml) at a 12.5 ppm level and a 5 min contact time in phosphate buffered saline at room temperature. Effects are related to a control (without bactericide) and to CTAB. G+ and G- denote Gram positive and Gram negative species, respectively.

TABLE 2

| Microorganisms (Type) | log (cfu/mol) | | |
|---|---|---|---|
| | control | betaine ester | CTAB |
| Bacillus megaterium (G+) | 6.7 | <3.3 | <3.3 |
| E. Coli (G-) | 7.0 | <3.3 | <3.3 |
| Candida albicans (yeast) | 6.2 | 6.2 | 5.7 |
| Pseudomonas aeruginosa (G-) | 7.4 | 7.5 | 7.5 |
| Staphylococcus aureus (G+) | 5.8 | <3.3 | <3.3 |
| Salmonella typhimurium (G-) | 5.5 | 3.9 | 3.8 |

The effects of the betaine ester and of CTAB are the same within experimental error. Except against the more resistant Ps. aeruginosa and the yeast C. albicans, high activities are found even at these low concentrations.

Example 3

The rate of alkaline hydrolysis of the present esters of betaines was determined for the compound in example 1 having totally 14 carbon atoms in its alkyl chain (p = 12). The conditions during the test and results obtained are given in the Table below.

T A B L E 3

Hydrolysis (given in %) at various pH values and reaction times at 30°C

| time hrs | pH 2.6 | 3 | 4 | 5 | 6 | 7 | 8 | 9 |
|---|---|---|---|---|---|---|---|---|
| 0.25 | | | | | | | | 96 |
| 0.5 | | | | | | | 12 | 100 |
| 1 | | | | | | 5 | 60 | 100 |
| 1.5 | | | | | | | 87 | |
| 2 | | | | | 2 | 10 | | |
| 3 | | | | | | | 100 | |
| 4 | | | | | | 37 | 100 | |
| 8 | <1 | <1 | 1 | | | 80 | | |
| 18 | | | | | 16 | | | |
| 20 | | | | | | 98 | | |
| 24 | <1 | 2 | 3 | 5 | | | | |
| 46 | | | | | 85 | | | |
| 48 | <1 | 2 | 5 | 10 | | | | |
| 120 | | | | | | | | |
| 144 | 1 | 6 | 12 | 22 | | | | |

The rate of hydrolysis is also temperature dependent. On a temperature increase from 25°C to 30°C the half-life at pH 7.0 is decreased from 9 to 5 hrs.

Example 4

The rate of hydrolysis and thereby the duration of the biological activity of the esters can also be regulated according to this invention by the salt concentration of the medium. The rate of hydrolysis is decreased considerably by an increase in sodium chloride concentration. In the Table below the effect of sodium chloride on hydrolysis of the compound in Example 2 at pH 7.9 and 30°C is shown.

TABLE 4

| | Hydrolysis, % | | | |
|---|---|---|---|---|
| | Molarity of NaCl | | | |
| t, hrs | 0 | 0.1 | 0.5 | 1.0 |
| 1 | 51 | 9 | 6 | 2 |
| 2 | 95 | 56 | 9 | 4 |
| 5 | 100 | 100 | 28 | 12 |
| 48 | | | 95 | 77 |

Example 5

A solution of 150 ppm of the compound in Example 1 in 10 mM phsophate buffer, pH 8.0, was analyzed at different points of time with respect to degree of hydrolysis as well as bactericidal effect against E. coli (NCTC 10418). The degree of hydrolysis was determined by analysis of the liberated 1-tetradecanol by gas chromatography (packed 4 ft glass column with 3% SP-2100 on 80/100 Supelcoport; isotermally at 130°C). The 1-tetradecanol was isolated by careful extraction with hexane. Internal standardization was carried out by means of 1-hexadecanol. Bactericidal effect was determined by dilution of a 1 ml sample to 25 ppm with 5 ml of a sterile buffer followed by addition of 60 µl of the bacterial suspension. After 5 min at room temperature a 100-fold dilution and spreading on an agar plate using a Spiral Plater system was performed. The plates were incubated at 37°C for 20 hrs and counted. Data from the experiments are given in the Table below.

TABLE 5

Hydrolysis and the effect on the bacterial activity.

| Time, min | Hydrolysis, % | Viable counts, cfu/ml (control: $7.1 \times 10^6$) |
|---|---|---|
| 5 | | 0 |
| 23 | 3 | |
| 30 | | $4.1 \times 10^3$ |
| 64 | 25 | |
| 109 | | $3.0 \times 10^6$ |
| 118 | 55 | |
| 149 | 86 | |
| 158 | | $7.9 \times 10^6$ |
| 179 | 89 | |
| 199 | | $7.8 \times 10^6$ |
| 217 | 100 | |
| 238 | 100 | |

Example 6

The effect of the following ammonium compounds on gonococci at a concentration of 30 ppm in an 0.01 M citrate solution with a pH value of 6 was determined after 1 and 10 minutes. The following results were obtained.

| Compound | Gonococci log cfu/ml 0 min | 1 min | 10 min |
|---|---|---|---|
| $(CH_3)_3\overset{+}{N}CH_2COOC_{14}H_{29}\ Cl^-$ | $10^6$ | $< 10^1$ | $< 10^1$ |
| $(CH_3)_3NCH_2COOC_{16}H_{33}\ Cl^-$ | $10^6$ | $< 10^1$ | $< 10^1$ |
| $(\overset{+}{N}H_3C_4H_8)CH(\overset{+}{N}H_3)COOC_{16}H_{33}\ 2Cl^-$ | $10^6$ | $< 10^3$ | $< 10^1$ |

Exemple 7

The effect of $(CH_3)_3\ NCH_2COOC_{14}H_{29}\ Cl^-$ in an 0.01 M citrate solution on herpes simplex virus was examined. The concentration of the ammonium compound was 20 ppm. After 10 minutes, the concentration of virus had been reduced by a factor greater than $10^3$.

**Claims**

1. A prophylactic agent for controlling venereal diseases, **characterized** in that it has applied on it or contains as a microbicide an ammonium compound having the general formula

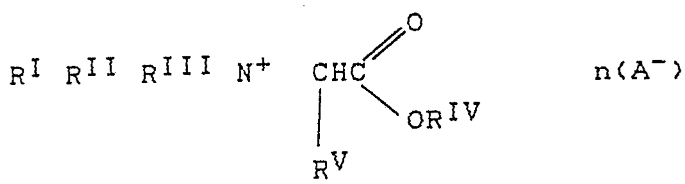

I

wherein $R^I$, $R^{II}$ and $R^{III}$ are hydrogen or lower alkyl groups and $R^{IV}$ is a long chain alkyl group having 10 to 18 carbon atoms, $R^V$ is hydrogen or a group having the formula $R^{VI}\ N^+H_3$, wherein $R^{VI}$ is an alkylen group having 3 to 4 carbon atoms, A is a monovalent counter ion, and n is the number of cationic groups in the compound.

2. An agent in accordance with claim 1, **characterized** in that the ammonium compound has the formula

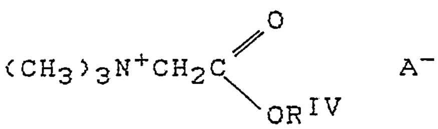

II

wherein $R^{IV}$ and A have the meanings stated above.

3. An agent in accordance with claim 1, **characterized** in that the ammonium compound has the formula

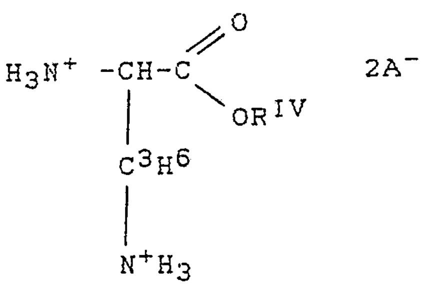

III

wherein $R^{IV}$ and A have the meanings stated above.

4. An agent in accordance with claim 1, **characterized** in that the ammonium compound has the formula

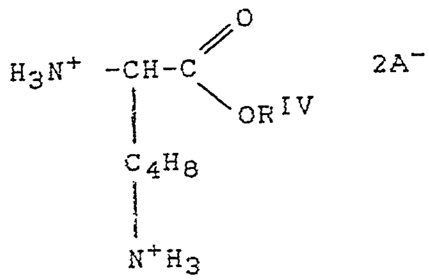

wherein $R^{IV}$ and A have the meanings stated above.

5. An agent in accordance with claims 1-4, **characterized** in that it has the shape of a condom or a vaginal pad.

6. An agent in accordance with claims 1-5, **characterized** in that the agent is a spray, an ointment, a foam or a gel.

7. The use of the agent in accordance with claims 1-6 for preventing the spreading of the diseases gonorrhea or chlamydia.

8. The use of the agent in accordance with claim 1-6 for preventing the spreading of the diseases herpes or aids.